# EUROPEAN PATENT SPECIFICATION

(11) **EP 1 123 710 B1**
(45) Date of publication and mention of the grant of the patent: **03.08.2005**
(21) Application number: 99947187.3
(22) Date of filing: 10.10.1999
(51) Int. Cl.: A61K 39/29

(54) **FREEZE-DRIED HEPATITIS A ATTENUATED LIVE VACCINE AND ITS STABILIZER**
GEFRIERGETROCKNETER, ATTENUIERTER HEPEATITIS-A-LEBENDIMPFSTOFF UND SEINE STABILISATOREN
VACCIN LYOPHILISE A VIRUS VIVANT MODIFIE D'HEPATITE A ET SON STABILISANT

(30) Priority: 19.10.1998 CN 98120633
(43) Date of publication of application: 16.08.2001
(73) Proprietor: Changchun Institute of Biological Products Ministry of Public Health, Changchun, Jilin Province 130062 (CN)
(72) Inventor: LIU, Jingye, Changchun, Jilin Province 130062 (CN); WANG, Pengfu, Changchun, Jilin Province 130062 (CN); LI, Guangpu, Changchun, Jilin Province 130062 (CN); XIE, Baosheng, Changchun, Jilin Province 130062 (CN); SONG, Zongming, Changchun, Jilin Province 130062 (CN); LI, Shuyan, Changchun, Jilin Province 130062 (CN); LIU, Jing, Changchun, Jilin Province 130062 (CN); YU, Ying, Changchun, Jilin Province 130062 (CN); ZHANG, Xizhen, Changchun, Jilin Province 130062 (CN); LIANG, Ben, Changchun, Jilin Province 130062 (CN); LIU, Lingjiu, Changchun, Jilin Province 130062 (CN); WANG, Wei, Changchun, Jilin Province 130062 (CN); ZHANG, Ling, Changchun, Jilin Province 130062 (CN); XUE, Yong, Changchun, Jilin Province 130062 (CN); LI, Jing, Changchun, Jilin Province 130062 (CN); LI, Yuhong, Changchun, Jilin Province 130062 (CN); LIN, Hui, Changchun, Jilin Province 130062 (CN); WAN, Zongju, Changchun, Jilin Province 130062 (CN)
(74) Representative: Lucas, Brian Ronald
(86) International application number: PCT/CN1999/000157
(87) International publication number: WO 2000/023104

(56) References cited:
- EP-A- 0 568 726
- EP-A- 1 129 723
- WO-A-98/28000
- CN-A- 1 191 490
- GB-A- 799 644
- JP-A- 1 279 843
- US-A- 5 075 110
- CHENG N.L. ET AL.: "Immunological effects of live attenuated hepatitis A vaccine." ZHONGHUA YIXUE ZAZHI, vol. 72, no. 10, October 1992 (1992-10), pages 581-583,638, XP001053214 & CN 1 076 726 A (CHANGCHUN) 29 September 1993 (1993-09-29)

## Description

### FIELD OF THE INVENTION

The present invention generally relates to attenuated hepatitis A vaccine, and more particularly to a stabilized lyophilized live hepatitis A vaccine formulation which can be preserved at ambient temperature for a long time, and so eliminate the pressures from transportation, storage and usage of the vaccine without loss of infectivity titers of the vaccine. The present invention further relates to a stabilizer for live lyophilized vaccine and its use in producing stabilized lyophilized live vaccine formulations.

### BACKGROUND OF THE INVENTION

Hepatitis A is a worldwide distributive acute disease caused by infection with hepatitis A virus (HAV) which is a picornavirus closely related to the poliovirus. Infection is spread by the fecal/oral route and consequently the disease in endemic in areas where hygiene and sanitation standards are lower. Recent reports on epidemical survey show that in developing countries including China, there are as many as 4 millions hepatitis A cases per year. There is frequently large-scale outbreak and rapid spread in certain regions with a poor social and economic status, especially after various disasters. In these countries or regions, as the high incidence of hepatitis A, some of serious public health and social problems have been encountered. On the other hand, in the United States and other developed countries, hepatitis A accounts for approximately 150,000 cases per year, that is approximately 25% of all clinical hepatitis cases.

Therefore, to successfully immunize against hepatitis A in developing countries as well as in developed countries, it is necessary to vaccinate the entire people, especially entire pediatric populations. So there will be an increasing need for hepatitis A vaccine.

An effective vaccine would be useful for active immunization of populations at high risk. Generally, there are four type of vaccine used for inducing a specific neutralizing antibody against challenge with virus or bacteria: live vaccine, inactivated vaccine, subunit vaccine (component vaccine), and recombinant vaccine. In these vaccines, the live attenuated vaccine could elicit a stronger protective response than others, and could have a significant impact on the eradication of the diseases.

U.S. Patent Nos.4, 532,215 and 4, 636,469 described, respectively, a strain of wild-type HAV, designated HM-175, initially isolated from feces of a patient, and adapted to passage in vitro in African green monkey kidney culture cell and methods for obtaining a vaccine by serial passaging. Also, CN Patent Nos.85107525 and 92114998 disclosed the preparations of attenuated HAV designated H₂ and L-A-1, respectively.

With regard to live attenuated hepatitis A vaccine, it worthily mentioned the live HAV vaccine based on strain CR-326F (Merck & Co. Inc.) which is under preclinical trials, and the vaccines based on strains H₂ and L-A-1, respectively, which have been licensed for practical use and industrial-scale production in China. Clinical serological studies demonstrated that these live attenuated hepatitis A vaccines, especially the vaccine prepared from L-A-1 strain of HAV (produced by Changchun Institute of Biological Products, Ministry of Public Health, Changchun, China) evoked high titers of antibody response in most of volunteers who received the vaccine after only one dose and no systemic complaints were present immediately after vaccination or during long -term follow-up (see CN Patent No.92114988).

However, all of these live hepatitis A vaccines used so far are in form of aqueous suspensions. One of the main disadvantage of live attenuated vaccine is not having satisfactory theremo-stability, even though in the situation of lyophilization at ambient temperature, hence it must be stored and transported under frozen state and used soon after thawing to insure effective vaccination. Hepatitis A virus as well as measles virus are unsatisfactory in both storage stability and heat resistance. For example, live attenuated hepatitis A virus survives only for about 7 days at a temperature of 2-8°C, and the valid storage-term is only about 3-6 months. Therefore, transportation and storage of these vaccine preparations must be finished at a reduced temperature (at a temperature of -20°C or lower, for example), that is so called "cold chain". As a direct result, the increases of production and transportation cost and user's expense are irrevocable, especially in developing countries and tropical and semitropical areas. So it would be an obstacle to popularization of the worldwide Expanded Program on Immunization (EPI) founded by World Health Organization (WHO).

For the reasons as described above , eradication of hepatitis A will depend on the ability to provide hepatitis A vaccine formulations having improved theremo-stability. So, there remains a distinct need in the art for live hepatitis vaccine formulations with enhanced storage stability and heat resistance during and after lyophilization.

### SUMMARY OF THE INVENTION

In view of these situations as mentioned above, the present inventors have made intensive studies during their production practices to overcome the problems mentioned above and to provide a lyophilized live hepatitis A vaccine with increased thermo-resistance and storage stability.The present inventors have surprisingly found that when a stabilizer solution is added to the vaccine stock suspension prepared by a disclosed method (a method described in CN Patent No.92114998, for example), and lyophilized, the hepatitis A vaccine formulation comprising, as a virus component, an attenuated live hepatitis A virus and a stabilizer comprising a high molecular weight structural additive, a disaccharide, a sugar alcohol, one or two amino acids and a buffering component, the storage-term of the hepatitis A virus-containing lyophilized vaccine is extended 3 times longer than non-treated stock viral suspension, so that the "cold chain" pressure and user's expense is decreased greatly to thereby increase the public acceptance of multitude of the vaccine imposed.

It is one object of the present invention to provide a stabilized lyophilized hepatitis A live vaccine formulation comprising a prophylactically effective viral titers of live attenuated hepatitis A virus and a stabilizer which can be preserved at ambient temperature for a long time, so that the "cold chain" pressures from transportation, storage and usage of the vaccine can be eliminated without loss of infective titers of the vaccine, to thereby greatly decrease the expense and relevant cost to ensure effective vaccination against hepatitis A.

In a preferred embodiment of this object of the present invention, said stock suspension of live attenuated hepatitis A virus is prepared by disclosed method from the wild-type HAV, strain L-A-I.

In a preferred embodiment of the present invention, said stabilizer for lyophilized live hepatitis A virus is composed of gelatin, trehalose, one or two amino acids selected from the group consisting of glutamic acid, aspartic acid, arginine, lysine or alkali metal salts thereof, ascorbic acid, urea, mannitol or sorbitol or both of them, and inositol.

According to a further preferred embodiment of the present invention, the stabilizer for lyophilized live virus vaccine may optionally contain human serum albumin.

In a further preferred embodiment of the stabilizer according to the present invention, the stabilizer for the lyophilized live virus is essentially composed of from 0 to 20 grams per liter of human serum albumin, from 5 to 10 grams per liter of gelatin, from 50 to 100 grams per liter of trehalose, from 7.5 to 15 grams per liter of sodium glutamate, from 0.5 to 5.5 grams per liter of ascorbic acid, from 5 to 28 grams per liter of urea, from 2 to 10 grams per liter of mannitol or sorbitol or a mixture of them, and from 4 to 10 grams per liter of inositol.

It is another object of the present invention to provide a method of preparing stabilized lyophilized live hepatitis A vaccine formulation as above , comprising:
(a) providing a stock suspension of attenuated live hepatitis A virus;
(b) adding a stabilizer solution to the stock suspension of step (a) at the ratio 1:1(v/v) to obtain a live vaccine formulation comprising prophylactically effective viral titers of live attenuated hepatitis A virus and a stabilizer for attenuated live virus, therein said stabilizer comprises gelatin, thehalose, one or two amino acid selected from the group consisting of glutamic acid, asparitc acid, arginine, lysine or alkali metal salts thereof, ascorbic acid, urea, mannitol or sorbitol or both of them, and inositol;
(c) lyophilizing said vaccine formulation obtained from the step (b).

According to a preferred embodiment of this object of the invention, wherein the lyophilization steps comprises precooling the vaccine formulation to from -20°C to -50°C over 3 to 6 hours, and then drying the live vaccine formulation by gradually increasing the temperature from -38°C to 35 °C in a lyophilizer.

It is a further object of the present invention to provide a stabilizer for lyophilized live virus, said stabilizer is essentially composed of gelatin, trehalose, one or two amino acids selected from the group consisting of glutamic acid, aspartic acid, arginine, lysine or alkali metal salts thereof, ascorbic acid, urea, mannitol or sorbitol or both of them, and inositol.

According to a preferred embodiment of this object of the present invention, wherein said stabilizer is essentially composed of from 0 to 20 grams per titer of human serum albumin, from 5 to 10 grams per liter of gelatin, from 50 to 100 grams per liter of trehalose, from 7.5 to 15 grams per liter of sodium glutamate, from 0.5 to 5.5 grams per liter of ascorbic acid, from 5 to 28 grams per liter of urea, from 2 to 10 grams per liter of mannitol or sorbital or a mixture of them, and from 4 to 10 gram per liter of inositol.

According to a further preferred embodiment of this object of the present invention, the stabilizer for lyophilized live virus vaccine may optionally contain human serum albumin.

According to a preferred embodiment of the present invention, said stabilizer is not only suitable for stabilizing lyophilized hepatitis A live virus, but also can be used for stabilizing viruses selected from the genus Enterovirus, against heat inactivation during the period of lyophilization and the period of storage and transportation post-lyophilization to ensure thermo-stability of the lyophilized live vaccine thereby to improve vaccination efficiency for susceptible population.

### DETAIL DESCRIPTION OF THE INVENTION

The present invention is related to lyophilized live vaccine formulations having a increased thermo-stability. Essentially, the vaccine formulations of the present invention are mixture of virus component and stabilizer components, therein the virus component comprises hepatitis A virus or at least one member selected from the genus Enterovirus, and the stabilizer components principally comprises gelatin, trehalose ,one or two amino acids selected from the group consisting of glutamic acid ,aspartic acid, arginine , lysine or alkali metal salts thereof, ascorbic acid ,urea, mannitol and/or sorbitol, and inositol. The vaccine stabilizer according to the present invention may optionally contain human serum albumin (HSA) in order to prevent any undesirable enzymolysis of the virus. Upon mixing the two components in a suitable ratio, result in a virus formulation which contains on a gram per liter of final vaccine formulations after lyophilization, from about 0 to 20 grams per liter of HSA, from 5 to 10 grams per liter of gelatin, from 50 to 100 grams per liter of trehalose, from 7.5 to 15 grams per liter of amino acid or alkali metal salts thereof, from 0.5 to 5.5 grams per liter of ascorbic acid, from 5 to 28 grams per liter of urea, from 2 to 10 grams per liter of mannitol or sorbitol or a mixture of them, and from 4 to 10 grams per liter of inositol.

With respect to an attenuated hepatitis A virus, for example, the HAV stock suspension used for the purpose of the present invention could be prepared from the wilde-type strain L-A-1 by the HAV cell-culture adaptation and attenuation method described in detail in CN Patent No.92114998. Briefly, the method comprises cultivating human diploid fibroblast cells in a suitable nutrient medium, for example Eagles's minimal essential medium(MEM) containing 10-15% fetal calf serum (FCS) in roller bottle at 37°C for 5-8 days. When confluent cell monolayers are formed, the cultured medium is discarded from a culturing vessel and the cells are washed with the same medium or PBS for 3 to 5 times. Then, these cells are inoculated with a seed virus of hepatitis A virus L-A-1 derived from human feces and purified by the method described in Example 1 of CN Patent No.92114998, and then the cells are cultivated in nutrient medium as above at 34-36°C for 3 to 4 weeks. After completion of the cultivation, changing the nutrient medium to medium 199 with or without phenol red, and cultivating cells at 34°C to 36°C for a additional 4 to 6 days in a cell roller. After harvesting, the cells are sonicated 3 times, then the cell debris is removed by centrifugation and collecting the resultant supernatant to obtain the desired stock suspension of the virus.

The present invention further provides a stabilizer advantageously used to stabilize a live vaccine , and to protect attenuated live virus against heat-inactivation at ambient temperature for a .long period, which is essentially composed of human serum albumin and/or gelatin, trehalose, one or two amino acid selected from the group consisting of glutamic acid, aspartic acid, arginine, lysine or alkali metal salts thereof, ascorbic acid, urea, mannitol and/or sorbitol, and inositol. In a particularly preferred embodiment of the present invention, a stabilizer contains about 0-20g/L of HSA, about 5-10g/L of gelatin, about 50-100g/L of trehalose, about 7.5-15g/:L of sodium glutamate, about 0.5-5.5g/L of ascorbic acid, about 5-28g/L of urea, about 2-10g/L of mannitol and /or sorbitol ,and 4-10g/L of insoitol.

Hepatitis A virus is a small picornavirus without outer envelope as well as any of other lipid. Like the majority of live enteroviruses, hepatitis A virus presented in form of aqueous suspension will rapidly loss their ability of replication or propagation, and infectious potency. In the absence of suitable stabilizer, it can not effectively protect against hepatitis A infection due to heat inactivation of the virus. Thus eradication of hepatitis A and other epidemic caused by infection with virus will depend on the ability to assure cold storage and transportation of virus vaccine. However, this problem has been circumvented by using vaccine formulations with improved stability according to the present invention.

Vaccine stabilizers are well known in the art as chemical compounds added to a vaccine formulation to enhance vaccine stability during period of the low temperature storage or lyophilization processing or storage post-lyophilization. As described above, the stabilizer aqueous solutions used for formulating and stabilizing the live vaccine of the present invention are preferably composed of a high molecular weight structural additive, a disaccharide, a sugar alcohol and water. The aqueous solution also includes one or two amino acids and buffering component. The combination of these components act to preserve the survival and activity of the virus upon freezing and lyophilization and a long storage period subsequent to lyophilization.

It is well known that the high molecular weight structural additive aids in preventing viral aggregation during freezing, and provides structural and nutritional support in the lyophilized or dried state. Within the context of the present invention, the preferred high molecular weight structural additives are human serum albumin and/or gelatin. The amino acids and sugar alcohols function to further preserve viral infectivity upon cooling and thawing of the aqueous suspension. In addition, these components function to preserve viral infectivity during sublimation of cooled aqueous virus suspension during lyophilization and in lyophilized state, and to contribute some buffer capacity. The preferred amino acids are arginine and glutamate, and the preferred sugar alcohols are mannitol, sorbitol and inositol. The trehalose, as a preferred disaccharide used in the stabilizer aqueous solution, could be immensely beneficial for stabilizing protein structure of virus to increase heat-resistance and for restoring vigorousness of virus after dehydration. Urea and ascorbic acid, except for the components mentioned above, play an important part in stabilizing hydration state or in maintaining osmotic balance during dehydration period. The buffering component acts to buffer the formulation by maintaining a relatively constant pH which preferably at about 7.0. The preferred buffer is balanced salt solution or PBS used for dissolving the chmical componds as mentioned above.

These components are added in substantially increasing amount to generate a vaccine stabilizer for combination with viral stock suspensions to thereby generate vaccine formulation for lyophilization which result in a increase in thermo-stability. The preferred component ranges disclosed in this specification allow for generation of vaccine formulations which, among other characteristics, exhibit improved thermo-stability over vaccine formulations known in the art.

The stabilizer of live attenuated vaccine can be formulated by a conventional method, for example by mixing each of components in a suitable vessel, except for preheating the mixture solution of trehalose, gelatin, urea, mannitol and /or sorbitol at about 37°C for 24 to 48 hours before adding HSA thereto. After 0.5 to 2 hours, mixing the resultant stabilizer with viral stock at about 1:1 (v/v) ratio.

It is noteworthy that the ranges of virus stabilizer and final vaccine formulation are presented on a gram per liter basis of the final vaccine formulation. One of ordinary skill in the art will be well aware that changing volume ratio of stabilizer to vaccine may be applied to practice the claimed invention, which in turn will require changes to the concentration of stabilizer components. Therefore, the invention not only limited to the specified 1:1 stabilizer/virus combination to generate the final vaccine formulation for lyophylization.

After the vaccine is formulated with stabilizer and viral stock, the resultant aqueous suspension should be dried by lyophilization cycles. Briefly, lyophilization cycle involves the steps of precooling the aqueous suspension below the gas transition temperature or below the eutectic point temperature (below -30°C) of the aqueous suspension for about 3 to 6 hours, and then removing water from the cooled suspension by sublimation to form a lyophilized virus. Within one preferred embodiment, aliquots of the formulated attenuated live virus are placed into a refrigerated chamber attached to a freeze dryer. A multistep freeze drying procedure is used to lyophilize the formulated live vaccine. The temperature is gradually increased from about -38°C to about 35°C over a period of 10 to 20 hours.

In order to demonstrate improvement in thermal stability and storage stability of live vaccine, the present invention is exemplified by viral potencies, for example by detecting viral titers pre- and post-lyophilization of hepatitis A vaccine, to observe the effectiveness of a stabilizer on the storage stability of live vaccine. The results show that the stabilizer included in the vaccine formulations of the present invention at a concentration sufficient to stabilize the live virus vaccine against heat inactivation remarkably improved thermal stability of the virus which have been lyophilized and incubated at 37 °C for one week as measured by the log CCID₅₀, as compared with lyophilized control vaccine formulation which is absence of stabilizer.

Further, the present inventors in a comparison experiment found that a similar result can be observed when a stabilizer solution which is minus human serum albumin (HSA) component is used for these live virus vaccine mentioned as above if the lyophilization cycle parameters could be suitable adjusted.

The following examples are provided to further illustrate the present invention. It is to be understood, however, that the examples are not to limit scope of the present invention.

### EXAMPLE 1

### Preparation of stabilizer (I) for lyophilized live virus

Following components are utilized for formulating the stabilizer solution (I):

| Component | Amounts (g/L) |
|---|---|
| Human Serum Albumin | 10.0 |
| Gelatin | 5.5 |
| Trehalose | 65.0 |
| Sodium Glutamate | 10.0 |
| Urea | 20.0 |
| Ascorbic Acid | 5.5 |
| Sorbitol | 6.6 |
| Inositol | 7.5 |

300 ml of distilled water is added to 5.5 g of purified gelatin, and the resultant mixture is heated for 40 minutes in an autoclave (116°C) to thereby obtain a solution. Cooling the solution to a temperature of about 30-35°C, and 10.0 g of HSA which has been sterilized by a series of ultrafiltration is added thereto. In accordance with the formulation as above, corresponding amount of trehalose, sodium glutamate, urea, ascorbic acid, sorbitol and inositol are added in this order, to 500ml of distilled water and thoroughly mixed. Then, the resultant solution is heated for 24 hours at 37°C. After cooling the solution to ambient temperature (about 22-26°C), put the solution into gelatin-HSA solution as above and mixing them thoroughly. Subsequently, distilled water is added thereto to make the total volume to 1000 ml. The pH of the resultant mixture solution is adjusted to about 7.0 by 0.1 N HCL, and is subjected to filtration sterilization once again, to obtain a stabilizer solution(I) which could be used for stabilizing live virus that to be lyophilized and stored.

### EXAMPLE 2

### Preparation of stabilizer(II) for lyophilized live vaccine

Following components are utilized for formulating the stabilizer solution(II) in accordance with the present invention, by a similar procedure as described in example 1.

| Component | Amount(g/L) |
|---|---|
| Gelatin | 8.5 |
| Trehalose | 75.0 |
| Urea | 15.5 |
| L-arginine | 10.1 |
| Ascorbic acid | 3.0 |
| Sorbitol | 5.0 |
| Mannitol | 5.0 |
| Inositol | 4.0 |

Except that HSA is eliminated from the components because it is very expensive and may cause virus contamination which derived from collected blood sources, and the sodium glutamate is replaced by arginine or alkali metal salt thereof, and added a small amount of inositol thereto.

### EXAMPLE 3

### Preparation of stabilized lyophilized hepatitis A live vaccine

Essentially, the stock suspension of hepatitis A live vaccine can be prepared by the method described in detail in CN Patent No. 92114998. Briefly, propagating human fetal lung diploid fibroblast cell infected with HAV strain L-A-1 in appropriate virus infectious dose in minimum essential medium (MEM) containing 10 % fetal calf serum (FCS) at 37°C for 3 to 4 weeks. When the positive infected cells are achieved more than 90 % as detected by indirect immunofluorescence, the nutrient medium is discarded from culturing vessel, and the residual FCS is washed away by phosphate-buffered saline (PBS). Then, the cultured medium replaced by medium 199 without phenol red therein, and the infected cells are cultivated for a additional 4 to 6 days. After completion of the culturing and collecting the infected cells by low-speed centrifugation, the infected cells are disrupted by means of 3 cycles of freeze-thawing and sonification. Cellular debris are removed by centrifugation to collect a supernatant as a stock material of the vaccine. The stock materiel (suspernatant product) gave a positive result as antigen by indirect immunofluorescence assay.

After the hepatitis A vaccine is formulated by mixing the stabilizer solution(I) prepared in Example 1 and viral stock suspension as above at about 1:1 (v/v) ratio, the resultant vaccine formulation is divided in a small volume (0.5 ml) into 2 ml glass vial. And then the aliquots of the formulated viral vaccine are placed into a freeze dryer (model FS150-SS20C, Hull Co, USA) for multistep lyophilization cycle at -40°C for 4 hours at first, then the shelf temperature is gradually increased to about -30°C and maintained primary drying. The shelf temperature is then gradually increased to 32°C and maintained there for 15 hours to thereby obtain desired lyophilized stabilized hepatitis A vaccine formulation with a very low moisture content.

### EXAMPLE 4

### Preparation of stabilized lyophilized measles live vaccine

The stock suspension of attenuated live measles vaccine is prepared in accordance with the Requirement for Measles Vaccine, Live in Chinese Requirements for Biological Products. The stock material is mixed with the stabilizer solution (II) prepared in Example 2 at 1:1 (v/v) ratio to obtain measles live vaccine formulation. The vaccine formulation is precooled at
-40°C for 5 hours, and then the formulation is subjected a drying treatment at about -35°C to 34°C for 14 hours to result in the lyophilized stabilized measles live vaccine.

### EXAMPLE 5

### Storage stability testing of lyophilized hepatitis A live vaccine

The samples of lyophilized hepatitis A live vaccine from different lots of viral formulation prepared in Example 3 which are stored at 2-8°C for 3 to 12 months, are ten-fold serially diluted, then the sample at 10⁻² to 10⁻⁷ dilution is used for detecting the viral titers every three months. The vaccine formulation from the same lot and lyophilized by the same lyophilization cycle parameters but no stabilizer therein is used as a control sample. After adding distilled water for injection to the lyophilized vaccine for reconstitution, the resultant aqueous suspention containing the live virus and stabilizer is subjected to testing for storage-stability by determing viral titers (CCID₅₀) using conventional enzyme linked immunosorbent assay (ELISA) and indirect-immunofluorescence assay (IF). The results of the testing reveal that the 5 lots of samples which are lyophilized in the presence of stabilizer solution exhibited a higher infectious titers in the range from about 6.33 to about 6.50 log CCID₅₀, whereas the 5 lots of control samples which are lyophilized in the absence of stabilizer solution exhibited a remarkable decreased infectious titers in the range from about 1.33 to 2.33 log CCID₅₀.

In another experiment, the lyophilized virus samples from same lot of vaccine formulations are stored at 2-8°C, 25°C and 37°C respectively, and each of the samples are sampled everyday and subjected to testing for storage stability in terms of lowest valid storage periods by detecting the CCID₅₀ values. Live vaccine in form of aqueous suspension is compared to lyophilized vaccine formulation.

The results are summarized in Table 1 and Table 2 below, respectively.

**Table 1**

| Storage stability test of lyophilized live hepatitis A vaccine formulation with stabilizer | | | | | |
|---|---|---|---|---|---|
| Lots | Months of storing at 2 -8 °C | | | | |
| | 0 | 3 | 6 | 9 | 12 |
| 1 | 6.50* | 6.67 | 6.67 | 6.50 | 6.50 |
| 2 | 6.67 | 6.50 | 6.67 | 6.50 | 6.67 |
| 3 | 6.50 | 6.50 | 6.33 | 6.50 | 6.50 |
| 4 | 6.50 | 6.67 | 6.67 | 6.50 | 6.33 |
| 5 | 6.33 | 6.50 | 6.50 | 6.50 | 6.33 |
| control | 2.33 | 1.75 | 1.50 | 1.50 | 1.33 |

| | | | | | |
|---|---|---|---|---|---|
| *Infective titers of the virus (log CCID₅₀/ml). | | | | | |

**Table 2**

| Comparison of stability of hepatitis A live vaccine | | |
|---|---|---|
| Temperature of storage | Lowest valid storage period (days) | |
| | Aqueous suspension | Lyophilized formulation |
| 2-8°C | 180 | 360 |
| 25°C | 7 | 90 |
| 37°C | 1 | 7 |

It can be seen from the results showed in table 1 and table 2 as above, that the stabilizer for lyophilized live vaccine of the present invention greatly increased thermo-stability measured as the log CCID₅₀ due to it stabilizes structure of viral protein and nucleic acid, and effectively maintains infectious potency of the vaccine under the conditions of enhanced temperature and osmotic strength.

### EXAMPLE 6

### The Immunogenicity and Safety Testing of the Lyophilized Hepatitis A Live Vaccine

The lyophilized hepatitis A live vaccine formulation prepared in accordance with the method in Example 1 which has been stored at about 25 °C for 30 days is intravenously inoculated into healthy rhesus monkeys (each group comprising 5 animals). Every two weeks bled for 8 weeks and checked for abnormally elevated serum enzymes (GPT) levels and the titers of anti-HAV antibody. Abnormal elevations of enzymes (more than 25U/ml) would indicate the presence of hepatitis A disease in the animals and the presence of antibody would show protection (Table 3). In this experiment, a fresh vaccine preparation in initial state from the same lot but which is unlyophilized and without stabilizers therein is used as a control sample. All of the animals are received a 10^{6.5} CCID₅₀ viral infectious dose (1.0 ml of the stock). The results are summarized in Table 3 below.

**Table 3**

| Serum GPT abnormal elevation and antibody response of animals before and after inoculation with the lyophilized hepatitis A vaccine | | | | | | | | | | | | |
|---|---|---|---|---|---|---|---|---|---|---|---|---|
| Lots | Abnormal elevation of SGPT* | | | | anti-HAV IgG Ab | | | | anti-HAV IgM Ab | | | |
| | 0 | 2 | 4 | 8(w) | 0 | 2 | 4 | 8(w) | 0 | 2 | 4 | 8(w) |
| 1 | 0/5 | 0/5 | 0/5 | 0/5 | 0** | 60 | 100 | 100 | 0** | 60 | 40 | 0 |
| 2 | 0/5 | 0/5 | 0/5 | 0/5 | 0 | 40 | 100 | 100 | 0 | 80 | 40 | 0 |
| 3 | 0/5 | 0/5 | 0/5 | 0/5 | 0 | 60 | 80 | 100 | 0 | 60 | 40 | 20 |
| 4 | 0/5 | 0/5 | 0/5 | 0/5 | 0 | 60 | 60 | 100 | 0 | 80 | 20 | 0 |

| | | | | | | | | | | | | |
|---|---|---|---|---|---|---|---|---|---|---|---|---|
| * Serum GPT value ≥ 25 U/ml is considered to be abnormal elevation of the enzyme. | | | | | | | | | | | | |
| ** The data given in the table represent percentage seroconvertion rates for 5 animals. | | | | | | | | | | | | |

It is can be seen from the results showed in Table 3, that all rhesus monkeys developed anti-HAV protective antibody and more than 80% of seroconverted animals also developed IgM anti-HAV at about two weeks after inoculation. In the other hand, none of the rhesus monkeys have elevation of liver enzymes atributable to the vaccination. All values for these higher primates is within normal limits. It indicates no biochemical evidence of hepatitis. These results exhibited comparable immunogenicity and safety with control samples, and show that the lyophilized live vaccine formulation of the present invention which has been stored for 30 days at ambient temperature still maintains a similar immunogenicity and safety like in its initial state.

### EXAMPLE 7

### Storage stability of lyophilized measles live vaccine

Storage-stability testing of measles live vaccine pro- and post-lyophilization stored at 2-8°C and 37°C respectively, is performed in substantially the same manner as described in Example 5.

The results show that, in the presence of stabilier of the present invention, 5 lots of the measles vaccine exhibited a slightly decreased themo-stability subsequent to lyophilization, that is less than 0.5 log loss in comparison to the control vaccine in its initial state. Further, the lyophilized measles vaccine sample which is stored at 2-8°C for 15 months and at 37°C for 4 weeks lossed their CCID₅₀ are less than 0.5 and 1.0, respectively.

## Claims

1. A stabilized lyophilized hepatitis A live vaccine formulation comprising a prophylactically effective viral titer of live attenuated hepatitis A virus and a stabilizer for lyophilized live hepatitis A virus, said stabilizer comprising a high molecular weight structural additive, a disaccharide, a sugar alcohol, one or two amino acids and a buffering component.

2. The stabilized lyophilised hepatitis A live vaccine formulation according to claim 1, wherein said live attenuated hepatitis A virus is prepared from the wild-type HAV, strain L-A-1.

3. The stabilized lyophilised hepatitis A live vaccine formulation according to claim 1, wherein said stabilizer for lyophilised live hepatitis A virus comprises human serum albumin or gelatin or both of them, trehalose, one or two amino acids selected from the group consisting of glutamic acid, aspartic acid, arginine, lysine or alkali metal salts thereof, ascorbic acid, urea, mannitol or sorbitol or both, and inositol.

4. The stabilized lyophilised hepatitis A live vaccine formulation according to claim 3, wherein said stabilizer for lyophilised live hepatitis A virus comprises from 0 to 20 grams per liter of human serum albumin, from 5 to 10 grams per liter of gelatin, from 50 to 100 grams per liter of trehalose, from 7.5 to 15 grams per liter of sodium glutamate, from 0.5 to 5.5 grams per liter of ascorbic acid, from 5 to 28 grams per liter of urea, from 2 to 10 grams per liter of mannitol or sorbitol, and from 4 to 10 grams per liter of inositol.

5. A method of preparing a stabilized lyophilised hepatitis A live vaccine formulation according to any one of the claims I to 4, comprising:
a. providing a stock suspension of attenuated live hepatitis A virus;
b. adding a stabilizer solution to a stock suspension of attenuated live hepatitis A virus at the ratio 1:1(v/v) to obtain a live vaccine formulation comprising prophylactically effective viral titers of live attenuated hepatitis A virus and a stabilizer for the lyophilised live virus, wherein said stabilizer comprises gelatin, trehalose, one or two amino acids selected from the group consisting of glutamic acid, aspartic acid, arginine, lysine or alkali metal salts thereof, ascorbic acid, urea, mannitol or sorbitol or a mixture of them, and inositol;
c. lyophilising said vaccine formulation obtained from the step (b).

6. The method according to claim 5, wherein the lyophilization step comprises precooling the vaccine formulation to from -20°C to -50°C for 3 to 6 hours, then drying the live vaccine formulation by gradually increasing the temperature from -38°C to 35°C in a lyophilizer.

7. A stabilizer for a lyophilised hepatitis A virus vaccine which comprises human serum albumin or gelatin or both of them, trehalose, one or two amino acids selected from the group consisting of glutamic acid, aspartic acid, arginine, lysine or alkali metal salts thereof, ascorbic acid, urea, mannitol or sorbitol or both, and inositol.

8. The stabilizer according to claim 7, wherein said stabilizer comprises from 0 to 20 grams per liter of human serum albumin, from 5 to 10 grams per liter of gelatin, from 50 to 100 grams per liter of trehalose, from 7.5 to 15 grams per liter of sodium glutamate, from 0.5 to 5.5 grams per liter of ascorbic acid, from 5 to 28 grams per liter of urea, from 2 to 10 grams per liter of mannitol or sorbitol, and from 4 to 10 grams per liter of inositol.

9. The stabilizer according to claim 7 for use in stabilizing a lyophilised live virus selected from genus Enterovirus.

## Patentansprüche

1. Rezeptur einer stabilisierten, gefriergetrockneten Hepatitis A lebend Vakzine umfassend einen vorbeugend wirkenden viralen Titer eines abgeschwächten lebend Hepatitis A Virus und einen Stabilisator für den gefriergetrockneten lebend Hepatis A Virus, wobei der Stabilisator einen hochmolekulares strukturierendes Additiv, ein Disaccharid, einen Zuckeralkohol, ein oder zwei Aminosäuren und eine Pufferkomponente umfasst.

2. Rezeptur der stabilisierten, gefriergetrockneten Hepatitis A lebend Vakzine nach Anspruch 1 **dadurch gekennzeichnet, dass** der abgeschwächte lebend Hepatitis A Virus vom wild Typ HAV, Stamm L-A-1, gewonnen wird.

3. Rezeptur der stabilisierten, gefriegetrockneten Hepatitis A lebend Vakzine nach Anspruch 1 **dadurch gekennzeichnet, dass** der Stabilisator für den gefriergetrockneten lebend Hepatits A Virus humanes Serumalbumin, Gelatine oder beide, Trehalose, ein oder zwei Aminosäuren, die Glutaminsäure, Asparaginsäure, Arginin, Lysin oder deren Alkaliemetallsalze sein können, Ascorbinsäure, Harnstoff, Mannitol, Sorbitol oder beide, sowie Inositol umfasst.

4. Rezeptur der stabilisierten, gefriergetrockneten Hepatitis A lebend Vakzine nach Anspruch 3, **dadurch gekennzeichnet, dass** der Stabilisator für den gefriergetrockneten lebend Hepatits A Virus 0 bis 20 g pro Liter humanes Serumalbumin, 5 bis 10 g pro Liter Gelatine, 50 bis 100 g pro Liter Trehalose, 7.5 bis 15 g pro Liter Natriumglutamat, 0.5 bis 5.5 g pro Liter Ascorbinsäure, 5 bis 28 g pro Liter Harnstoff, 2 bis 10 g pro Liter Mannitol oder Sorbitol und 4 bis 10 g pro Liter Inositol, umfasst.

5. Methode zur Herstellung einer stabilisierten, gefriergetrockneten Hepatitis A lebend Vakzin-Rezeptur nach Ansprüchen 1 bis 4, umfassend:
a. die Bereitstellung einer Stocksuspension des abgeschwächten lebend Hepatitis A Virus;
b. Hinzugeben der Stabilisatorlösung zur Stocksuspension des abgeschwächten lebend Hepatitis A Virus in einem Volumenverhältnis von 1 : 1, um eine Rezeptur einer lebend Vakzin zu erhalten umfassend einen vorbeugend wirkenden viralen Titer eines abgeschwächten lebend Hepatis A Virus und einen Stabilisator für den gefriergetrockneten lebend Hepatis A Virus, wobei der Stabilisator Gelatine, Trehalose, ein oder zwei Aminosäuren, die Glutaminsäure, Asparaginsäure, Arginin, Lysine oder deren Alkalimetallsalze sein können, Ascorbinsäure, Harnstoff, Mannitol, Sorbitol oder eine Mischung aus diesen beiden, sowie Insonitol umfasst;
c. Gefriertrocknung der aus Schritt (b.) erhaltenen Vakzin-Rezeptur.

6. Methode nach Anspruch 5, **dadurch gekennzeichnet, dass** der Gefriertrocknungsschritt ein 3 bis 6 stündiges Vorkühlen der Vakzin-Rezeptur auf -20 °C bis -50°C und eine anschließende Trocknung der lebend Vakzin-Rezeptur in einem Lyophilisator, wobei die Temperatur schrittweise von -38°C bis 35°C erhöht wird, umfasst.

7. Stabilisator einer gefriergetrockneten Hepatitis A Virus Vakzine umfassend humanes Serumalbumin, Gelatine oder beides; Trehalose; einoder zwei Aminosäuren, die Glutaminsäure, Asparaginsäure, Arginin, Lysin oder deren Alkalimetallsalze sein können; Ascorbinsäure; Harnstoff; Mannitol, Sorbitol oder beides, sowie Inositol.

8. Stabilisator nach Anspruch 7, **dadurch gekennzeichnet, dass** der Stabilisator 0 bis 20 g pro Liter humanes Serumalbumin, 5 bis 10 g pro Liter Gelatine, 50 bis 100 g pro Liter Trehalose, 7.5 bis 15 g pro Liter Natriumglutamat, 0.5 bis 5.5 g pro Liter Ascorbinsäure, 5 bis 28 g pro Liter Harnstoff, 2 bis 10 g pro Liter Mannitol oder Sorbitol und 4 bis 10 g pro Liter Inositol umfasst.

9. Stabilisator nach Anspruch 7 zur Verwendung zur Stabilisierung eines gefriergetrockneten lebend Virus ausgewählt aus der Gattung der Enteroviren.

## Revendications

1. Formulation de vaccin vivant contre l'hépatite A lyophilisée et stabilisée comprenant un titre viral prophylactiquement efficace du virus vivant et atténué de l'hépatite A et un stabilisant pour le virus vivant lyophilisé de l'hépatite A, ledit stabilisant comprenant un additif structurel à masse moléculaire élevée, un disaccharide, un alcool de sucre, un ou deux acides aminés et un composant tampon.

2. Formulation de vaccin vivant contre l'hépatite A lyophilisée et stabilisée selon la revendication 1, dans laquelle ledit virus vivant et atténué de l'hépatite A est préparé à partir du HAV de type sauvage, souche L-A-1.

3. Formulation de vaccin vivant contre l'hépatite A lyophilisée et stabilisée selon la revendication 1, dans laquelle ledit stabilisant pour le virus vivant lyophilisé de l'hépatite A comprend de la sérumalbumine humaine ou de la gélatine ou les deux, du tréhalose, un ou deux acides aminés choisis dans le groupe formé de l'acide glutamique, l'acide aspartique, l'arginine, la lysine ou les sels de métaux alcalins de celle-ci, l'acide ascorbique, l'urée, le mannitol ou le sorbitol ou les deux, et l'inositol.

4. Formulation de vaccin vivant contre l'hépatite A lyophilisée et stabilisée selon la revendication 3, dans laquelle ledit stabilisant pour le virus vivant lyophilisé de l'hépatite A comprend 0 à 20 grammes par litre de sérumalbumine humaine, 5 à 10 grammes par litre de gélatine, 50 à 100 grammes par litre de tréhalose, 7,5 à 15 grammes par litre de glutamate de sodium, 0,5 à 5,5 grammes par litre d'acide ascorbique, 5 à 28 grammes par litre d'urée, 2 à 10 grammes par litre de mannitol ou de sorbitol et 4 à 10 grammes par litre d'inositol.

5. Procédé de préparation d'une formulation de vaccin vivant contre l'hépatite A lyophilisée et stabilisée selon l'une quelconque des revendications 1 à 4, comprenant les étapes consistant à :
a. fournir une réserve d'une suspension du virus vivant et atténué de l'hépatite A ;
b. ajouter une solution stabilisante à la réserve d'une suspension du virus vivant et atténué de l'hépatite A dans un rapport 1:1 (v/v) pour obtenir une formulation de vaccin vivant comprenant des titres viraux prophylactiquement efficaces du virus vivant et atténué de l'hépatite A et un stabilisant pour le virus vivant lyophilisé, dans lequel ledit stabilisant comprend de la gélatine, du tréhalose, un ou deux acides aminés choisis dans le groupe formé de l'acide glutamique, de l'acide aspartique, de l'arginine, de la lysine ou des sels de métaux alcalins de celle-ci, de l'acide ascorbique, de l'urée, du mannitol ou du sorbitol ou un mélange de ceux-ci, et de l'inositol ;
c. lyophiliser ladite formulation de vaccin obtenue à l'étape (b).

6. Procédé selon la revendication 5, dans lequel l'étape de lyophilisation comprend les étapes consistant à préalablement refroidir la formulation de vaccin de -20°C à -50°C pendant 3 à 6 heures puis à sécher la formulation de vaccin vivant en augmentant graduellement la température de -38°C à 35°C dans un lyophilisateur.

7. Stabilisant pour un vaccin du virus de l'hépatite A lyophilisé qui comprend de la sérumalbumine humaine ou de la gélatine ou les deux, du tréhalose, un ou deux acides aminés choisis dans le groupe formé de l'acide glutamique, l'acide aspartique, l'arginine, la lysine ou des sels de métaux alcalins de celle-ci, l'acide ascorbique, l'urée, le mannitol ou le sorbitol ou les deux, et l'inositol.

8. Stabilisant selon la revendication 7, dans lequel ledit stabilisant comprend 0 à 20 grammes par litre de sérumalbumine humaine, 5 à 10 grammes par litre de gélatine, 50 à 100 grammes par litre de tréhalose, 7,5 à 15 grammes par litre de glutamate de sodium, 0,5 à 5,5 grammes par litre d'acide ascorbique, 5 à 28 grammes par litre d'urée, 2 à 10 grammes par litre de mannitol ou de sorbitol et 4 à 10 grammes par litre d'inositol.

9. Stabilisant selon la revendication 7 pour une utilisation dans la stabilisation d'un virus vivant lyophilisé choisi dans le genre Entérovirus.
